(19) Europäisches Patentamt
European Patent Office
Office européen des brevets

(11) EP 0 656 929 B1

(12) EUROPÄISCHE PATENTSCHRIFT

(45) Veröffentlichungstag und Bekanntmachung des Hinweises auf die Patenterteilung:
**05.02.1997 Patentblatt 1997/06**

(21) Anmeldenummer: **93915892.9**

(22) Anmeldetag: **13.07.1993**

(51) Int Cl.[6]: **C10L 1/10**

(86) Internationale Anmeldenummer:
**PCT/EP93/01830**

(87) Internationale Veröffentlichungsnummer:
**WO 94/02570 (03.02.1994 Gazette 1994/04)**

# (54) VERWENDUNG VON IM IR-BEREICH ABSORBIERENDEN UND/ODER FLUORESZIERENDEN VERBINDUGEN ALS MARKIERUNGSMITTEL FÜR FLÜSSIGKEITEN

USE OF COMPOUNDS WHICH ABSORB AND/OR FLUORESCE IN THE I/R RANGE AS MARKERS FOR LIQUIDS

UTILISATION DE COMPOSES A ABSORPTION ET/OU FLUORESCENCE DANS LE DOMAINE IR, COMME AGENTS DE MARQUAGE DE SUBSTANCES LIQUIDES

(84) Benannte Vertragsstaaten:
**AT BE CH DE DK ES FR GB GR IE IT LI NL PT SE**

(30) Priorität: **23.07.1992 DE 4224301**
**23.12.1992 DE 4243776**
**23.12.1992 DE 4243774**

(43) Veröffentlichungstag der Anmeldung:
**14.06.1995 Patentblatt 1995/24**

(73) Patentinhaber: **BASF Aktiengesellschaft**
**67063 Ludwigshafen (DE)**

(72) Erfinder:
- **ALBERT, Bernhard**
  **D-6701 Maxdorf (DE)**
- **KIPPER, Juergen**
  **D-7500 Karlsruhe 1 (DE)**
- **VAMVAKARIS, Christos**
  **D-6701 Kallstadt (DE)**
- **BECK, Karin, Heidrun**
  **D-6700 Ludwigshafen (DE)**
- **WAGENBLAST, Gerhard**
  **D-6719 Weisenheim (DE)**

(56) Entgegenhaltungen:
**EP-A- 0 155 780**
**EP-A- 0 192 215**
**EP-A- 0 310 080**
**EP-A- 0 358 080**
**EP-A- 0 464 543**
**US-A- 3 484 467**
**US-A- 4 009 008**
**US-A- 4 209 302**
**US-A- 4 735 631**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 656 929 B1

## Beschreibung

Die vorliegende Erfindung betrifft die Verwendung von Verbindungen aus der Klasse der metallfreien oder metallhaltigen Phthalocyanine, der metallfreien oder metallhaltigen Naphthalocyanine, der Nickel-Dithiolen-Komplexe,der Aminiumverbindungen von aromatischen Aminen, der Methinfarbstoffe oder der Azulenquadratsäurefarbstoffe, die ihr Absorptionsmaximum im Bereich von 600 bis 1 200 nm und/oder ein Fluoreszenzmaximum im Bereich von 620 bis 1 200 nm aufweisen, als Markierungsmittel für Flüssigkeiten, ein Verfahren zur Detektion von Markierungsmittel in Flüssigkeiten sowie einen dazu geeigneten Detektor.

Es ist häufig erforderlich, Flüssigkeiten zu markieren, um in der Folge, z.B. bei ihrer Anwendung, mittels geeigneter Methoden die so markierten Flüssigkeiten wieder zu detektieren.

Beispielsweise kann auf diese Weise Heizöl von Dieselöl unterschieden werden.

Aufgabe der vorliegenden Erfindung war es nun, geeignete Verbindungen, die sich als Markierungsmittel eignen, bereitzustellen. Die Markierungsmittel sollten im nahen Infrarot eine ausreichend starke Absorption und/oder Fluoreszenz aufweisen, so daß die Detektion der Absorption mit üblichen Photometern, die in diesem Bereich empfindlich sind, und/oder der Fluoreszenz mit üblichen Geräten, nach Anregung mit einer geeigneten Strahlenquelle, erfolgen kann.

Demgemäß wurde gefunden, daß sich die eingangs näher bezeichneten Verbindungen vorteilhaft als Markierungsmittel eignen.

Metallhaltige Phthalocyanine oder Naphthalocyanine weisen in der Regel Lithium (zweimal), Magnesium, Zink, Kupfer, Nickel, VO, TiO oder AlCl als Zentralatom auf.

Geeignete Phthalocyanine gehorchen z.B. der Formel Ia

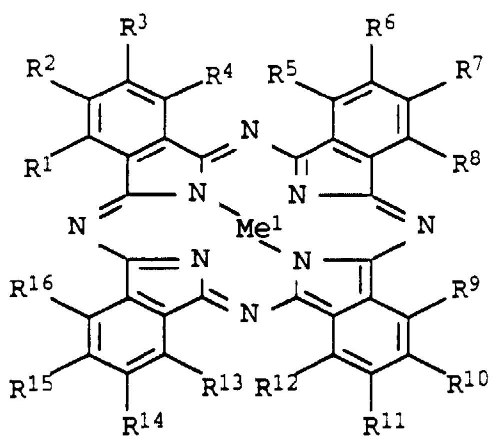

(Ia),

in der

$Me^1$ zweimal Wasserstoff, zweimal Lithium, Magnesium, Zink, Kupfer, Nickel, VO, TiO, AlCl oder $Si(OH)_2$,

mindestens 4 der Reste $R^1$ bis $R^{16}$ unabhängig voneinander einen Rest der Formel $W\text{-}X^1$, worin W für eine chemische Bindung, Schwefel, Imino, $C_1$-$C_4$-Alkylimino oder Phenylimino und $X^1$ für $C_1$-$C_{20}$-Alkyl, das gegebenenfalls durch 1 bis 4 Sauerstoffatome in Etherfunktion unterbrochen ist und durch Phenyl substituiert sein kann, Adamantyl oder gegebenenfalls substituiertes Phenyl stehen, und

gegebenenfalls die übrigen Reste $R^1$ bis $R^{16}$ Wasserstoff, Halogen, Hydroxysulfonyl oder $C_1$-$C_4$-Dialkylsulfamoyl bedeuten.

Geeignete Phthalocyanine gehorchen weiterhin z.B. der Formel Ib

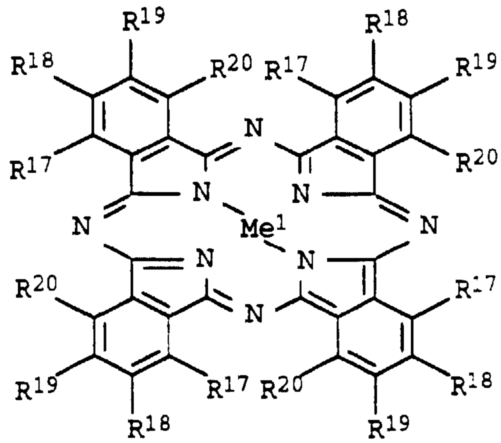

(Ib),

in der

$R^{17}$ und $R^{18}$ oder $R^{18}$ und $R^{19}$ oder $R^{19}$ und $R^{20}$ zusammen jeweils einen Rest der Formel $X^2$-$C_2H_4$-$X^3$, worin einer der beiden Reste $X^2$ und $X^3$ für Sauerstoff und der andere für Imino oder $C_1$-$C_4$-Alkylimino steht, und

$R^{19}$ und $R^{20}$ oder $R^{17}$ und $R^{20}$ oder $R^{17}$ und $R^{18}$ unabhängig voneinander jeweils Wasserstoff oder Halogen bedeuten und

$Me^1$ die obengenannte Bedeutung besitzt.

Geeignete Naphthalocyanine gehorchen z.B. der Formel II

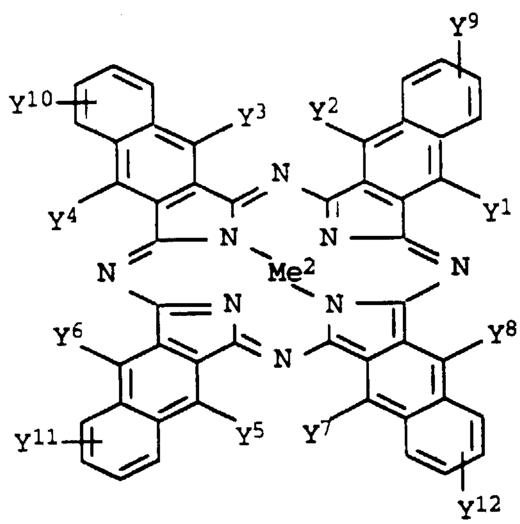

(II),

in der

$Y^1$, $Y^2$, $Y^3$, $Y^4$, $Y^5$, $Y^6$, $Y^7$ und $Y^8$ unabhängig voneinander jeweils Wasserstoff, Hydroxy, $C_1$-$C_{20}$-Alkyl oder $C_1$-$C_{20}$-Alkoxy, wobei die Alkylgruppen jeweils durch 1 bis 4 Sauerstoffatome in Etherfunktion unterbrochen sein können und gegebenenfalls durch Phenyl substituiert sind,

$Y^9$, $Y^{10}$, $Y^{11}$ und $Y^{12}$ unabhängig voneinander jeweils Wasserstoff, $C_1$-$C_{20}$-Alkyl oder $C_1$-$C_{20}$-Alkoxy, wobei die Alkylgruppen jeweils durch 1 bis 4 Sauerstoffatome in Etherfunktion unterbrochen sein können, Halogen, Hydroxysulfonyl oder $C_1$-$C_4$-Dialkylsulfamoyl und

$Me^2$ zweimal Wasserstoff, zweimal Lithium, Magnesium, Zink, Kupfer, Nickel, VO, TiO, AlCl oder den Rest

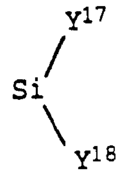

bedeuten, wobei

$Y^{17}$ und $Y^{18}$ unabhängig voneinander jeweils für Hydroxy, $C_1$-$C_{20}$-Alkoxy, $C_1$-$C_{20}$-Alkyl, $C_2$-$C_{20}$-Alkenyl, $C_3$-$C_{20}$-Alkenyloxy oder einen Rest der Formel

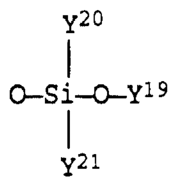

stehen, worin $Y^{19}$ die Bedeutung von $C_1$-$C_{20}$-Alkyl, $C_2$-$C_{20}$-Alkenyl oder $C_4$-$C_{20}$-Alkadienyl und $Y^{20}$ und $Y^{21}$ unabhängig voneinander jeweils die Bedeutung von $C_1$-$C_{12}$-Alkyl, $C_2$-$C_{12}$-Alkenyl oder des obengenannten Rests $OY^{19}$ besitzen.

Von besonderem Interesse sind dabei Naphthalocyanine der Formel II, in der mindestens einer der Reste $Y^1$ bis $Y^8$ von Wasserstoff verschieden sind.

Geeignete Nickel-Dithiolen-Komplexe gehorchen z.B. der Formel III

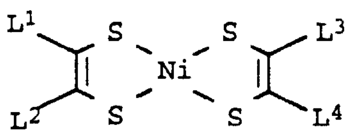

(III),

in der

$L^1$, $L^2$, $L^3$ und $L^4$ unabhängig voneinander jeweils $C_1$-$C_{20}$-Alkyl, das gegebenenfalls durch 1 bis 4 Sauerstoffatome in Etherfunktion unterbrochen ist, Phenyl, $C_1$-$C_{20}$-Alkylphenyl, $C_1$-$C_{20}$-Alkoxyphenyl, wobei die Alkylgruppen jeweils durch 1 bis 4 Sauestoffatome in Etherfunktion unterbrochen sein können, oder $L^1$ und $L^2$ und/oder $L^3$ und $L^4$ jeweils zusammen den Rest der Formel

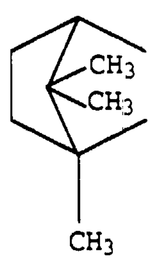

bedeuten.

Geeignete Aminiumverbindungen gehorchen z.B. der Formel IV

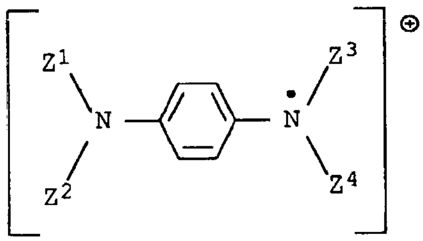 An$^{\ominus}$ (IV),

in der
$Z^1$, $Z^2$, $Z^3$ und $Z^4$ unabhängig voneinander jeweils $C_1$-$C_{20}$-Alkyl, das gegebenenfalls durch 1 bis 4 Sauerstoffatome in Etherfunktion unterbrochen ist, $C_1$-$C_{20}$-Alkanoyl oder einen Rest der Formel

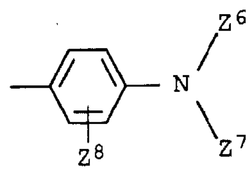 ,

worin $Z^6$ für Wasserstoff, $C_1$-$C_{20}$-Alkyl, das gegebenenfalls durch 1 bis 4 Sauerstoffatome in Etherfunktion unterbrochen ist, oder $C_1$-$C_{20}$-Alkanoyl, $Z^7$ für Wasserstoff oder $C_1$-$C_{20}$-Alkyl, das gegebenenfalls durch 1 bis 4 Sauerstoffatome in Etherfunktion unterbrochen ist, und $Z^8$ für Wasserstoff, $C_1$-$C_{20}$-Alkyl, das gegebenenfalls durch 1 bis 4 Sauerstoffatome in Etherfunktion unterbrochen ist, oder Halogen stehen, und
An$^{\ominus}$ das Äquivalent eines Anions bedeuten.

Geeignete Methinfarbstoffe gehorchen z.B. der Formel V

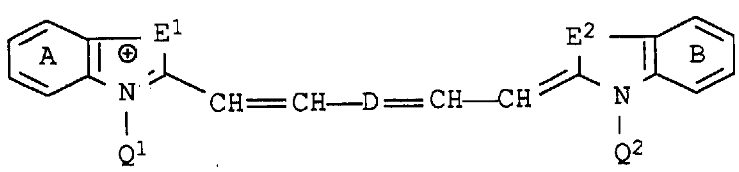 n An$^{\ominus}$ (V),

in der die Ringe A und B unabhängig voneinander jeweils gegebenenfalls benzoanelliert sind und substituiert sein können,
$E^1$ und $E^2$ unabhängig voneinander jeweils Sauerstoff, Schwefel, Imino oder einen Rest der Formel

$-C(CH_3)_2-$ oder $-CH=CH-$,

D einen Rest der Formel

$-CE^3=$ oder $-CH=CE^3-CH=$,

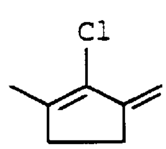 oder 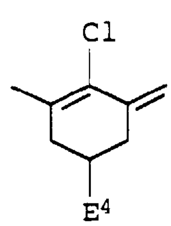 ,

worin $E^3$ für Wasserstoff, $C_1$-$C_6$-Alkyl, Chlor oder Brom und $E^4$ für Wasserstoff oder $C_1$-$C_6$-Alkyl stehen,

$Q^1$ und $Q^2$ unabhängig voneinander jeweils Phenyl, $C_5$-$C_7$-Cycloalkyl, $C_1$-$C_{12}$-Alkyl das durch 1 bis 3 Sauerstoffatome in Etherfunktion unterbrochen sein kann und gegebenenfalls durch Hydroxy, Chlor, Brom, Carboxyl, $C_1$-$C_4$-Alkoxycarbonyl, Acryloyloxy, Methacryloyloxy, Hydroxysulfonyl, $C_1$-$C_7$-Alkanoylamino, $C_1$-$C_6$-Alkylcarbamoyl, $C_1$-$C_6$-Alkylcarbamoyloxy oder einen Rest der Formel $G^{\oplus}(K)_3$, worin G für Stickstoff oder Phosphor und K für Phenyl, $C_5$-$C_7$-Cycloalkyl oder $C_1$-$C_{12}$-Alkyl stehen, substituiert sind,

$An^{\ominus}$ das Äquivalent eines Anions und

n 1, 2 oder 3 bedeuten.

Geeignete Azulenquadratsäurefarbstoffe gehorchen z.B. der Formel VI

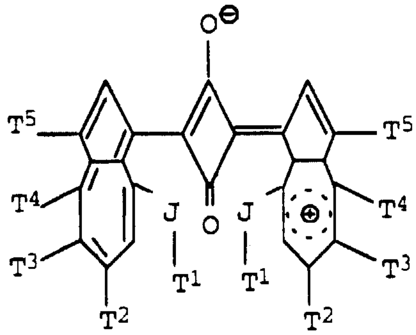

(VI),

in der

J $C_1$-$C_{12}$-Alkylen,

$T^1$ Wasserstoff, Halogen, Amino, Hydroxy, $C_1$-$C_{12}$-Alkoxy, Phenyl, substituiertes Phenyl, Carboxyl, $C_1$-$C_{12}$-Alkoxycarbonyl, Cyano oder einen Rest der Formel -$NT^7$-CO-$T^6$, -CO-$NT^6T^7$ oder O-CO-$NT^6T^7$, worin $T^6$ und $T^7$ unabhängig voneinander jeweils für Wasserstoff, $C_1$-$C_{12}$-Alkyl, $C_5$-$C_7$-Cycloalkyl, Phenyl, 2,2,6,6-Tetramethylpiperidin-4-yl oder Cyclohexylaminocarbonyl stehen, und

$T^2$, $T^3$, $T^4$ und $T^5$ unabhängig voneinander jeweils Wasserstoff oder $C_1$-$C_{12}$-Alkyl, das gegebenenfalls durch Halogen, Amino, $C_1$-$C_{12}$-Alkoxy, Phenyl, substituiertes Phenyl, Carboxyl, $C_1$-$C_{12}$-Alkoxycarbonyl oder Cyano substituiert ist, bedeuten,

mit der Maßgabe, daß wenn $T^5$ Wasserstoff bedeutet, an einem oder beiden Azulenringen die Ringpositionen der Substituenten J-$T^1$ und $T^4$ innerhalb eines Azulenrings auch gegeneinander vertauscht sein können.

Alle in den obengenannten Formeln auftretenden Alkyl-, Alkylen- oder Alkenylreste können sowohl geradkettig als auch verzweigt sein.

In Formel Ia, II, III oder IV sind geeignete $C_1$-$C_{20}$-Alkylreste, die gegebenenfalls durch 1 bis 4 Sauerstoffatome in Etherfunktion unterbrochen sind, z.B. Methyl, Ethyl, Propyl, Isopropyl, Butyl, Isobutyl, sec-Butyl, tert-Butyl, Pentyl, Isopentyl, Neopentyl, tert-Pentyl, Hexyl, 2-Methylpentyl, Heptyl, Octyl, 2-Ethylhexyl, Isooctyl, Nonyl, Isononyl, Decyl, Isodecyl, Undecyl, Dodecyl, Tridecyl, 3,5,5,7-Tetramethylnonyl, Isotridecyl (die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436), Tetradecyl, Pentadecyl, Hexadecyl, Heptadecyl, Octadecyl, Nonadecyl, Eicosyl, 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 2- oder 4-Butoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 4,8-Dioxadecyl, 3,6,8-Trioxadecyl, 3,6,9-Trioxaundecyl, 3,6,9,12-Tetraoxatridecyl oder 3,6,9,12-Tetraoxatetradecyl.

In Formel I oder II ist geeignetes $C_1$-$C_{20}$-Alkyl, das durch Phenyl substituiert ist, z.B. Benzyl oder 1- oder 2-Phenylethyl.

In Formel II, III oder IV sind geeignete $C_1$-$C_{20}$-Alkoxyreste, die gegebenenfalls durch 1 bis 4 Sauerstoffatome in Etherfunktion unterbrochen sind, z.B. Methoxy, Ethoxy, Propoxy, Isopropoxy, Butoxy, Isobutoxy, Pentyloxy, Hexyloxy, Heptyloxy, Octyloxy, 2-Ethylhexyloxy, Isooctyloxy, Nonyloxy, Isononyloxy, Decyloxy, Isodecyloxy, Undecyloxy, Dodecyloxy, Tridecyloxy, Isotridecyloxy, Tetradecyloxy, Pentadecyloxy, Hexadecyloxy, Heptadecyloxy, Octadecyloxy, Nonadecyloxy, Eicosyloxy, 2-Methoxyethoxy, 2-Ethoxyethoxy, 2-Propoxyethoxy, 2-Isopropoxyethoxy, 2-Butoxyethoxy, 2-oder 3-Methoxypropoxy, 2- oder 3-Ethoxypropoxy, 2- oder 3-Propoxypropoxy, 2- oder 3-Butoxypropoxy, 2- oder 4-Methoxybutoxy, 2-oder 4-Ethoxybutoxy, 2- oder 4-Propoxybutoxy, 2- oder 4-Butoxybutoxy, 3,6-Dioxaheptyloxy, 3,6-Dioxaoctyloxy, 4,8-Dioxanonyloxy, 3,7-Dioxaoctyloxy, 3,7-Dioxanonyloxy, 4,7-Dioxaoctyloxy, 4,7-Dioxanonyloxy, 4,8-Dioxadecyloxy, 3,6,8-Trioxadecyloxy, 3,6,9-Trioxaundecyloxy, 3,6,9,12-Tetraoxatridecyloxy oder 3,6,9,12-Tetraoxatetradecyloxy.

In Formel II ist geeignetes $C_1$-$C_{20}$-Alkoxy, das durch Phenyl substituiert ist, z.B. Benzyloxy oder 1- oder 2-Phenylethoxy.

In Formel Ia, III oder VI ist geeignetes substituiertes Phenyl z.B. durch $C_1$-$C_6$-Alkyl, $C_1$-$C_6$-Alkoxy, Hydroxy oder Halogen substituiertes Phenyl. In der Regel können dabei 1 bis 3 Substituenten auftreten.

Halogen in Formel Ib, II, IV oder VI ist z.B. Fluor, Chlor oder Brom.

Reste W in Formel Ia sowie $X^2$ oder $X^3$ in Formel Ib sind z.B. Methylimino, Ethylimino, Propylimino, Isopropylimino oder Butylimino.

Reste $R^1$ bis $R^{16}$ in Formel Ia sowie $Y^9$ bis $Y^{12}$ in Formel II sind z.B. Dimethylsulfamoyl, Diethylsulfamoyl, Dipropylsulfamoyl, Dibutylsulfamoyl oder N-Methyl-N-ethylsulfamoyl.

$C_2$-$C_{20}$-Alkenyl sowie $C_4$-$C_{20}$-Alkandienyl in Formel II ist z.B. Vinyl, Allyl, Prop-1-en-1-yl, Methallyl, Ethallyl, But-3-en-1-yl, Pentenyl, Pentadienyl, Hexadienyl, 3,7-Dimethylocta-1,6-dien-1-yl, Undec-10-en-1-yl, 6,10-Dimethylundeca-5,9-dien-2-yl, Octadec-9-en-1-yl, Octadeca-9,12- dien-1-yl, 3,7,11,15-Tetramethylhexadec-1-en-3-yl oder Eicos-9-en-1-yl.

$C_3$-$C_{20}$-Alkenyloxy in Formel II ist z.B. Allyloxy, Methallyloxy, But-3-en-1-yloxy, Undec-10-en-1-yloxy, Octadec-9-en-1-yloxy oder Eicos-9-en-1-yloxy.

$Z^6$ in Formel IV bedeutet z.B. Formyl, Acetyl, Propionyl, Butyryl, Isobutyryl, Pentanoyl, Hexanoyl, Heptanoyl, Octanoyl oder 2-Ethylhexanoyl.

Wenn die Ringe A und/oder B in Formel V substituiert sind, so können als Substituenten z.B. $C_1$-$C_6$-Alkyl, Phenyl-$C_1$-$C_6$-alkoxy, Phenoxy, Halogen, Hydroxy, Amino, $C_1$-$C_6$-Mono- oder Dialkylamino oder Cyano in Betracht kommen. Die Ringe sind dabei in der Regel 1 bis 3-fach substituiert.

Reste $E^3$, $E^4$, $Q^1$ und $Q^2$ in Formel V sind z.B. Methyl, Ethyl, Propyl, Isopropyl, Butyl, Isobutyl, sec-Butyl, Pentyl, Isopentyl, Neopentyl, tert-Pentyl oder Hexyl.

Reste $Q^1$ und $Q^2$ sind weiterhin z.B. Hexyl, 2-Methylpentyl, Heptyl, Octyl, 2-Ethylhexyl, Isooctyl, Nonyl, Isononyl, Decyl, Isodecyl, Undecyl, Dodecyl, Cyclopentyl, Cyclohexyl, 2-Methoxyethyl, 2-Ethoxyethyl, 2- oder 3-Methoxypropyl, 2- oder 3-Ethoxypropyl, 2-Hydroxyethyl, 2- oder 3-Hydroxypropyl, 2-Chlorethyl, 2-Bromethyl, 2- oder 3-Chlorpropyl, 2- oder 3-Brompropyl, 2-Carboxyethyl, 2- oder 3-Carboxypropyl, 2-Methoxycarbonylethyl, 2-Ethoxycarbonylethyl, 2- oder 3-Methoxycarbonylpropyl, 2- oder 3-Ethoxycarbonylpropyl, 2-Acryloyloxyethyl, 2- oder 3-Acryloyloxypropyl, 2-Methacryloyloxyethyl, 2- oder 3-Methacryloyloxypropyl, 2-Hydroxysulfonylethyl, 2- oder 3-Hydroxysulfonylpropyl, 2-Acetylaminoethyl, 2- oder 3-Acetylaminopropyl, 2-Methylcarbamoylethyl, 2-Ethylcarbamoylethyl, 2- oder 3-Methylcarbamoylpropyl, 2- oder 3-Ethylcarbamoylpropyl, 2-Methylcarbamoyloxyethyl, 2-Ethylcarbamoyloxyethyl, 2- oder 3-Methylcarbamoyloxypropyl, 2- oder 3-Ethylcarbamoyloxypropyl, 2-(Trimethylammonium)ethyl, 2-(Triethylammonium)ethyl, 2- oder 3-(Trimethylammonium)propyl, 2- oder 3-(Triethylammonium)propyl, 2-(Triphenylphosphonium)ethyl oder 2- oder 3-(Triphenylphosphonium)propyl.

$An^{\ominus}$ in Formel IV oder V leitet sich z.B. von Anionen organischer oder anorganischer Säuren ab. Besonders bevorzugt sind dabei z.B. Methansulfonat, 4-Methylbenzolsulfonat, Acetat, Trifluoroacetat, Heptafluorobutyrat, Chlorid, Bromid, Iodid, Perchlorat, Tetrafluoroborat, Nitrat, Hexafluorophosphat oder Tetraphenylborat.

Reste J in Formel VI sind z.B. Methylen, Ethylen, 1,2- oder 1,3-Propylen, 1,2-, 1,3-, 2,3- oder 1,4-Butylen, Pentamethylen, Hexamethylen, Heptamethylen, Octamethylen, Nonamethylen, Decamethylen, Undecamethylen oder Dodecamethylen.

Reste $T^2$, $T^3$, $T^4$ und $T^5$ in Formel VI sind beispielsweise Methyl, Ethyl, Propyl, Isopropyl, Butyl, Isobutyl, sec-Butyl, tert-Butyl, Pentyl, Isopentyl, Neopentyl, tert-Pentyl, 2-Methylbutyl, Hexyl, 2-Methylpentyl, Heptyl, Octyl, 2-Ethylhexyl, Isooctyl, Nonyl, Isononyl, Decyl, Undecyl, Dodecyl, Fluormethyl, Chlormethyl, Difluormethyl, Trifluormethyl, Trichlormethyl, 2-Fluorethyl, 2-Chlorethyl, 2-Bromethyl, 1,1,1-Trifluorethyl, Heptafluorpropyl, 4-Chlorbutyl, 5-Fluorpentyl, 6-Chlorhexyl, Cyanomethyl, 2-Cyanoethyl, 3-Cyanopropyl, 2-Cyanobutyl, 4-Cyanobutyl, 5-Cyanopentyl, 6-Cyanohexyl, 2-Aminoethyl, 2-Aminopropyl, 3-Aminopropyl, 2-Aminobutyl, 4-Aminobutyl, 5-Aminopentyl, 6-Aminohexyl, 2-Hydroxyethyl, 2-Hydroxypropyl, 3-Hydroxypropyl, 2-Hydroxybutyl, 4-Hydroxybutyl, 5-Hydroxypentyl, 6-Hydroxyhexyl, 2-Methoxyethyl, 2-Ethoxyethyl, 2-Propoxyethyl, 2-Isopropoxyethyl, 2-Butoxyethyl, 2-Methoxypropyl, 2-Ethoxypropyl,

3-Ethoxypropyl, 4-Ethoxybutyl, 4-Isopropoxybutyl, 5-Ethoxypentyl, 6-Methoxyhexyl, Benzyl, 1-Phenylethyl, 2-Phenylethyl, 4-Chlorbenzyl, 4-Methoxybenzyl, 2-(4-Methylphenyl)ethyl, Carboxymethyl, 2-Carboxyethyl, 3-Carboxypropyl, 4-Carboxybutyl, 5-Carboxypentyl, 6-Carboxyhexyl, Methoxycarbonylmethyl, Ethoxycarbonylmethyl, 2-Methoxycarbonylethyl, 2-Ethoxycarbonylethyl, 3-Methoxycarbonylpropyl, 3-Ethoxycarbonylpropyl, 4-Methoxycarbonylbutyl, 4-Ethoxycarbonylbutyl, 5-Methoxycarbonylpentyl, 5-Ethoxycarbonylpentyl, 6-Methoxycarbonylhexyl oder 6-Ethoxycarbonylhexyl.

$T^1$ in Formel VI ist z.B. Methoxycarbonyl, Ethoxycarbonyl, Propoxycarbonyl, Isopropoxycarbonyl, Butoxycarbonyl, Isobutoxycarbonyl, sec-Butoxycarbonyl, tert-Butoxycarbonyl, Pentyloxycarbonyl, Isopentyloxycarbonyl, Neopentyloxycarbonyl, tert-Pentyloxycarbonyl, Hexyloxycarbonyl, Heptyloxycarbonyl, Octyloxycarbonyl, Isooctyloxycarbonyl, Nonyloxycarbonyl, Isononyloxycarbonyl, Decyloxycarbonyl, Isodecyloxycarbonyl, Undecyloxycarbonyl, Dodecyloxycarbonyl, Methoxy, Ethoxy, Propoxy, Isopropoxy, Butoxy, Isobutoxy, Pentyloxy, Hexyloxy, Acetylamino, Carbamoyl, Mono- oder Dimethylcarbamoyl, Mono- oder Diethylcarbamoyl, Monocyclohexylcarbonyl, Phenylcarbamoyl, Dimethylcarbamoyloxy oder Diethylcarbamoyloxy.

Bevorzugt ist die erfindungsgemäße Verwendung von solchen Verbindungen, die aus der Klasse der metallfreien oder metallhaltigen Naphthalocyanine stammen.

Hervorzuheben ist die erfindungsgemäße Verwendung von Naphthalocyaninen der Formel IIa

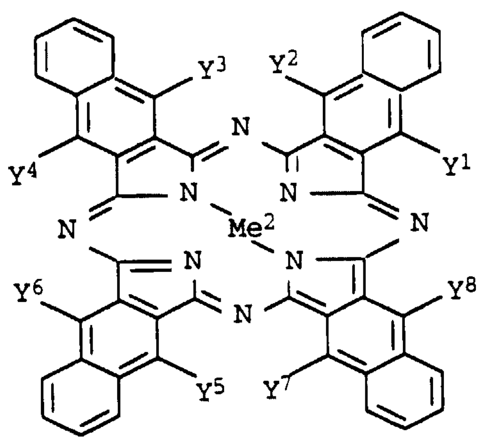

(IIa),

in der

$Y^1$, $Y^2$, $Y^3$, $Y^4$, $Y^5$, $Y^6$, $Y^7$ und $Y^8$ unabhängig voneinander jeweils Wasserstoff, Hydroxy, $C_1$-$C_4$-Alkyl oder $C_1$-$C_{20}$-Alkoxy und

$Me^2$ zweimal Wasserstoff, zweimal Lithium, Magnesium, Zink, Kupfer, Nickel, VO, AlCl oder den Rest

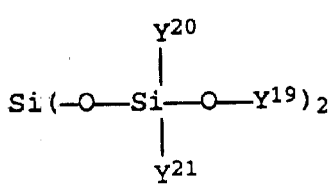

bedeuten, worin $R^{19}$ für $C_1$-$C_{13}$-Alkyl oder $C_{10}$-$C_{20}$-Alkadienyl und $Y^{20}$ und $Y^{21}$ unabhängig voneinander jeweils für $C_1$-$C_{13}$-Alkyl oder $C_2$-$C_4$-Alkenyl stehen.

Besonders hervorzuheben ist die erfindungsgemäße Verwendung von Naphthalocyaninen der Formel IIa, in der $Y^1$, $Y^2$, $Y^3$, $Y^4$, $Y^5$, $Y^6$, $Y^7$ und $Y^8$ unabhängig voneinander jeweils Hydroxy, $C_1$-$C_{20}$-Alkoxy, insbesondere $C_1$-$C_{10}$-Alkoxy bedeuten. Die Alkoxyreste können dabei gleich oder verschieden sein.

Besonders hervorzuheben ist weiterhin die erfindungsgemäße Verwendung von Naphthalocyaninen der Formel IIa, in der $Me^2$ zweimal Wasserstoff bedeutet.

Hervorzuheben ist weiterhin die erfindungsgemäße Verwendung von Nickel-Dithiolen-Komplexen der Formel III,

in der $L^1$, $L^2$, $L^3$ und $L^4$ unabhängig voneinander jeweils Phenyl, $C_1$-$C_{20}$-Alkylphenyl, $C_1$-$C_{20}$-Alkoxyphenyl oder durch Hydroxy und $C_1$-$C_{20}$-Alkyl substituiertes Phenyl oder $L^1$ und $L^2$ sowie $L^3$ und $L^4$ jeweils zusammen den Rest der Formel

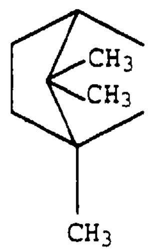

bedeuten.

Besonders hervorzuheben ist weiterhin die erfindungsgemäße Verwendung von Nickel-Dithiolen-Komplexen der Formel III, in der $L^1$ und $L^4$ jeweils Phenyl und $L^2$ und $L^4$ jeweils einen Rest der Formel 4-[$C_2H_5$-$C(CH_3)_2$]-$C_6H_4$ bedeuten.

Die Phthalocyanine der Formel Ia sind an sich bekannt und z.B. in DE-B-1 073 739 oder EP-A-155 780 beschrieben oder können nach an sich bekannten Methoden, wie sie bei der Herstellung von Phthalocyaninen oder Naphthalocyaninen zur Anwendung kommen und wie sie beispielsweise in F.H. Moser, A.L. Thomas "The Phthalocyanines", CRC Press, Boca Rota, Florida, 1983, oder J. Am. Chem. Soc. Band 106, Seiten 7404 bis 7410, 1984, beschrieben sind, erhalten werden. Die Phthalocyanine der Formel Ib sind ebenfalls an sich bekannt und z.B. in EP-A-155 780 beschrieben oder können gemäß den Methoden des obengenannten Standes der Technik (Moser, J.Am. Chem.Soc.) erhalten werden.

Die Naphthalocyanine der Formel II sind ebenfalls an sich bekannt und beispielsweise in der EP-A-336 213, EP-A-358 080, GB-A-2 168 372 oder GB-A-2 200 650 beschrieben oder können gemäß den Methoden des obengenannten Standes der Technik (Moser, J.Am. Chem.Soc.) erhalten werden.

Die Nickel-Dithiolen-Komplexe der Formel III sind ebenfalls an sich bekannt und beispielsweise in der EP-A-192 215 beschrieben.

Die Aminiumverbindungen der Formel IV sind ebenfalls an sich bekannt und z.B. in US-A-3 484 467 beschrieben oder können gemäß den dort genannten Methoden erhalten werden.

Die Methinfarbstoffe der Formel V sind ebenfalls an sich bekannt und z.B. in der EP-A-464 543 beschrieben oder können gemäß den dort genannten Methoden erhalten werden.

Die Azulenquadratsäurefarbstoffe der Formel VI sind ebenfalls an sich bekannt und z.B. in der EP-A-310 080 oder US-A-4 990 649 beschrieben oder können gemäß den dort genannten Methoden erhalten werden.

Geeignete Lösungsmittel, die erfindungsgemäß mittels der oben näher bezeichneten Verbindungen markiert werden können, sind insbesondere organische Flüssigkeiten, beispielsweise Alkohole, wie Methanol, Ethanol, Propanol, Isopropanol, Butanol, Isobutanol, sec-Butanol, Pentanol, Isopentanol, Neopentanol oder Hexanol, Glykole, wie 1,2-Ethylenglykol, 1,2- oder 1,3-Propylenglykol, 1,2-, 2,3- oder 1,4-Butylenglykol, Di- oder Triethylenglykol oder Di- oder Tripropylenglykol, Ether, wie Methyl-tertbutylether, 1,2-Ethylenglykolmono- oder -dimethylether, 1,2-Ethylenglykolmono- oder -diethylether, 3-Methoxypropanol, 3-Isopropoxypropanol, Tetrahydrofuran oder Dioxan, Ketone, wie Aceton, Methylethylketon oder Diacetonalkohol, Ester, wie Essigsäuremethylester, Essigsaureethylester, Essigsäurepropylester oder Essigsäurebutylester, aliphatische oder aromatische Kohlenwasserstoffe wie Pentan, Hexan, Heptan, Octan, Isooctan, Petrolether, Toluol, Xylol, Ethylbenzol, Tetralin, Dekalin, Dimethylnaphthalin, Testbenzin, Mineralöl, wie Benzin, Kerosin, Dieselöl oder Heizöl, natürliche Öle, wie Olivenöl, Sojaöl oder Sonnenblumenöl, oder natürliche oder synthetische Motoren-, Hydraulik- oder Getriebeöle, z.B. Fahrzeugmotorenöl oder Nähmaschinenöl, oder Bremsflüssigkeiten.

Besonders vorteilhaft verwendet man die obengenannten Verbindungen zum Markieren von Mineralölen, bei denen gleichzeitig eine Kennzeichnung gefordert wird, z.B. aus steuerlichen Gründen. Um die Kosten der Kennzeichnung gering zu halten, strebt man üblicherweise an, für die Färbung möglichst ausgiebige Farbstoffe zu verwenden. Jedoch sind selbst sogenannte farbstarke Farbstoffe in hoher Verdünnung in Mineralölen rein visuell nicht mehr wahrnehmbar.

Aus diesem Grund ist es von besonderem Vorteil, solche Markierstoffe zu verwenden, die ihr Absorptionsmaximum vom Bereich von 600 bis 1 200 nm aufweisen und/oder die im Bereich von 620 bis 1 200 nm fluoreszieren, da sie mit geeigneten Instrumenten leicht detektiert werden können.

Zum Markieren der Flüssigkeiten, insbesondere aber von Mineralöl, werden die obengenannten Verbindungen im allgemeinen in Form von Lösungen angewandt. Als Lösungsmittel eignen sich vorzugsweise aromatische Kohlenwasserstoffe, wie Toluol oder Xylol. Um eine zu hohe Viskosität der resultierenden Lösungen zu vermeiden, wählt man im allgemeinen eine Konzentration an IR-Strahlung absorbierender und/oder im IR-Bereich fluoreszierender Verbindung

von 2 bis 50 Gew.-%, bezogen auf die Lösung.

Ein weiterer Gegenstand der vorliegenden Erfindung ist ein Verfahren zur Detektion von Markierungsmitteln in Flüssigkeiten, das dadurch gekennzeichnet ist, daß die Markierungsmittel anhand ihrer Fluoreszenz im NIR-Spektralbereich (naher Infrarot-Spektralbereich) nachgewiesen werden.

Die Fluoreszenz der in den Flüssigkeiten enthaltenen Markierungsmittel wird vorteilhaft mit einem Halbleiterlaser oder einer Halbleiterdiode angeregt. Besonders günstig ist es, dabei einen Halbleiterlaser oder eine Halbleiterdiode mit einer Wellenlänge der maximalen Emission im Spektralbereich von $\lambda_{max}$ -100 nm bis $\lambda_{max}$ +20 nm anzuwenden. $\lambda_{max}$ bedeutet dabei die Wellenlänge des Absorptionsmaximums des Markierstoffs. Die Wellenlänge der maximalen Emission liegt dabei im Bereich von 620 bis 1 200 nm.

Das so erzeugte Fluoreszenzlicht wird vorteilhaft mit einem Halbleiterdetektor, insbesondere mit einer Silicium-Photodiode oder einer Germanium-Photodiode, detektiert.

Besonders vorteilhaft gelingt der Nachweis, wenn sich vor dem Detektor noch ein Interferenzfilter und/oder ein Kantenfilter (mit einer kurzwelligen Transmissionskante im Bereich von $\lambda_{max}$ bis $\lambda_{max}$ +80 nm) und/oder ein Polarisator befindet.

Mittels der obengenannten Verbindungen gelingt es sehr einfach, markierte Flüssigkeiten nachzuweisen, selbst wenn die Markierungssubstanzen nur in einer Konzentration von ungefähr 0,1 ppm (Nachweis durch Absorption) oder ungefähr 5 ppb (Nachweis durch Fluoreszenz) vorliegen.

Die vorliegende Erfindung betrifft weiterhin einen Detektor zur Ausübung des erfindungsgemäßen Verfahrens, wobei der Detektor eine NIR-Lichtquelle, ausgewählt aus einem Halbleiterlaser und einer Halbleiterdiode, ein oder mehrere optische Filter, einen NIR-Polarisator und einen Photodektor, ausgewählt aus einer Silicium-Photodiode und einer Germanium-Photodiode, sowie gegebenenfalls Lichtleitfasern oder -faserbündel enthält.

Die folgenden Beispiele sollen die Erfindung näher erläutern.

Allgemeine Vorschrift zum Nachweis der Markierungsmittel.

I. Nachweis durch Absorption im IR-Bereich

Beispiel 1

Es wurde soviel Farbstoff der Formel

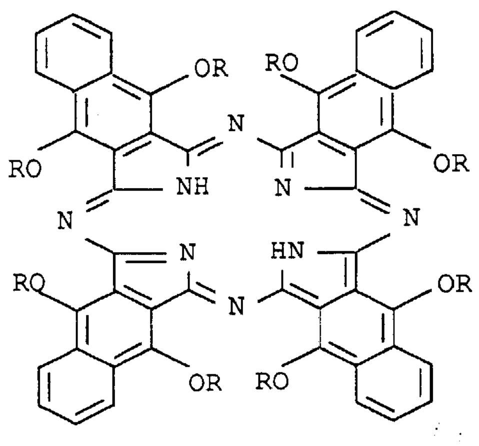

R=n-$C_4H_9$

in Dieselkraftstoff gelöst, daß eine Lösung mit einem Gehalt an Farbstoff von 1 000 ppm erhalten wurde.

Diese Lösung wurde stufenweise weiter verdünnt und ihre Absorption im NIR-Bereich im Vergleich mit reinem Dieselkraftstoff mittels eines handelsüblichen Spektrometers (1 cm-Küvette) vermessen.

| Farbstoffgehalt in Dieselkraftstoff [ppm] | Absorption | Absorptionsmaximum [nm] |
|---|---|---|
| 100 | >> 3 | - |
| 50 | 3,05 | 844,0 |
| 20 | 2,81 | 854,0 |
| 10 | 2,10 | 860,4 |

(fortgesetzt)

| Farbstoffgehalt in Dieselkraftstoff [ppm] | Absorption | Absorptionsmaximum [nm] |
|---|---|---|
| 1 | 0,27 | 860,0 |

Ähnlich günstige Ergebnisse werden erzielt, wenn man Naphthalocyanine der obengenannten Formel (mit R = n-$C_5H_{11}$ oder n-$C_{12}H_{25}$) oder die im folgenden aufgeführten Farbstoffe zum Markieren verwendet, wobei Nc jeweils den Rest eines Naphthalocyaninsystems bedeutet.

Farbstoff 2

Hexadecaphenylthio-kupferphthalocyanin

Farbstoff 3

Tetradecaphenylthio-kupferphthalocyanin

Farbstoff 4

Tetradecadodecylthio-kupferphthalocyanin

Farbstoff 5

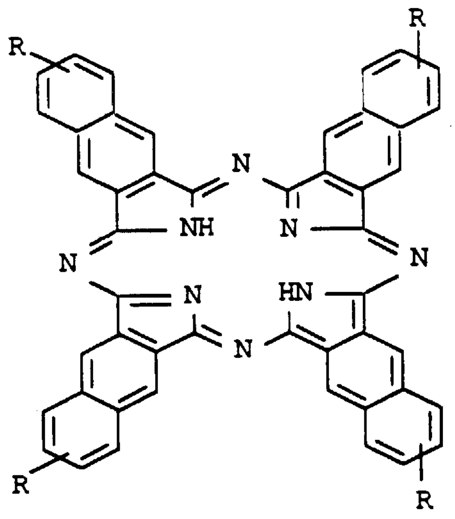

(R=t-$C_4H_9$)

Farbstoff 6

Hexadeca(4-tert-butylphenylthio)-kupferphthalocyanin

Farbstoff 7

$$NcSi[-O-Si(CH_3)_2-O-C_{12}H_{25}]_2$$

Farbstoff 8

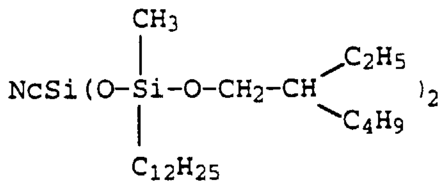

Farbstoff 9

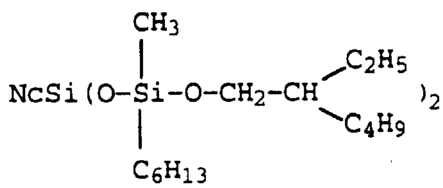

Farbstoff 10

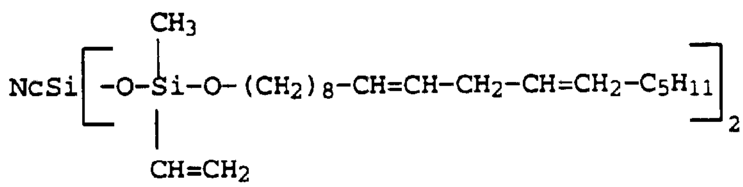

Farbstoffe 11 bis 15

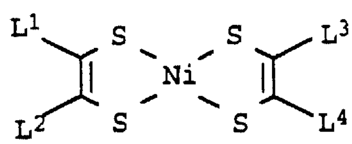

| Farbstoff-Nr. | $L^1$ | $L^2$ | $L^3$ | $L^4$ |
|---|---|---|---|---|
| 11 | 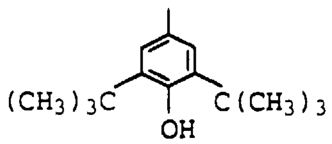 | $C_6H_5$ | 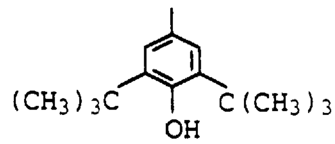 | $C_6H_5$ |
| 12 | 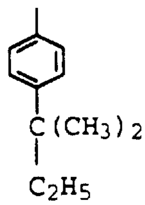 | $C_6H_5$ | 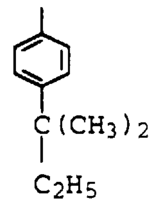 | $C_6H_5$ |
| 13 | $C_6H_5$ | $C_6H_5$ | $C_6H_5$ | $C_6H_5$ |
| 14 | 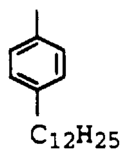 | $C_6H_5$ | 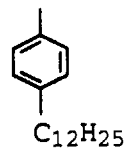 | $C_6H_5$ |
| 15 | 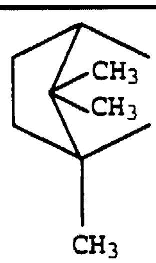 | | 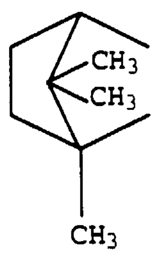 | |

Farbstoffe 15 bis 18

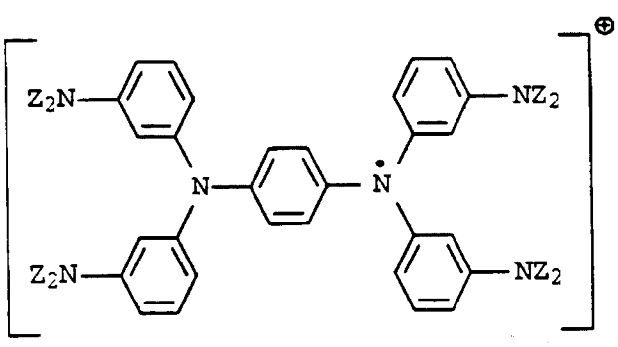

$An^{\ominus}$

| Farbstoff Nr. | Z | $An^{\ominus}$ |
|---|---|---|
| 16 | $C_4H_9$ | $NO_3^{\ominus}$ |

(fortgesetzt)

| Farbstoff Nr. | Z | An$^{\ominus}$ |
|---|---|---|
| 17 | $C_2H_5$ | $NO_3^{\ominus}$ |
| 18 | $C_4H_9$ | $BF_4^{\ominus}$ |

Farbstoffe 19 bis 23

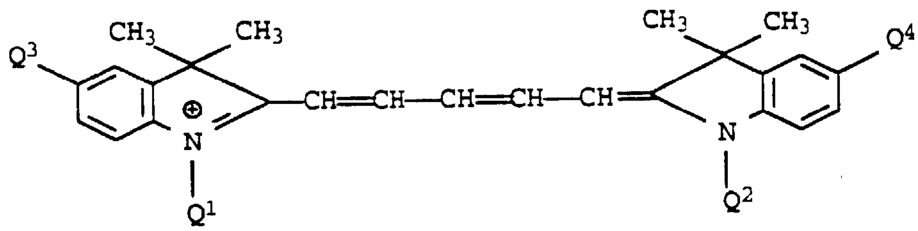

| Farbstoff Nr. | $Q^1$ | $Q^2$ | $Q^3$ | $Q^4$ | An$^{\ominus}$ |
|---|---|---|---|---|---|
| 19 | $CH_3$ | $CH_3$ | Cl | Cl | $I^{\ominus}$ |
| 20 | $CH_3$ | $CH_3$ | H | H | $I^{\ominus}$ |
| 21 | $C_2H_4O\overset{O}{\overset{\|}{C}}NHC(CH_3)_3$ | $C_2H_4O\overset{O}{\overset{\|}{C}}NHC(CH_3)_3$ | H | H | $I^{\ominus}$ |
| 22 | $C_2H_4\overset{O}{\overset{\|}{C}}NHC_6H_{13}$ | $C_2H_4\overset{O}{\overset{\|}{C}}NHC_6H_{13}$ | H | H | $ClO_4^{\ominus}$ |
| 23 | $C_3H_6SO_3^{\ominus}$ | $C_3H_6SO_3H$ | H | H | Betain |

Farbstoff Nr. 24

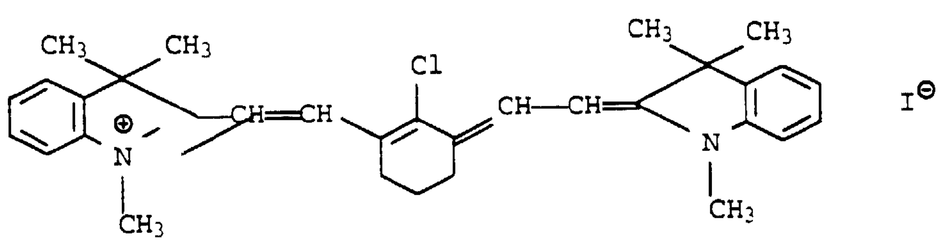

II. Nachweis durch Fluoreszenz im NIR-Bereich

Die Abbildung 1 zeigt den schematischen Aufbau des Detektors.

Zur Anregung der Marker-Fluoreszenz wird die Emission eines kommerziellen Halbleiterdiodenlasers benutzt. Der parallele Laserstrahl wird auf die in einer 1-cm-Küvette befindliche Probe gestrahlt. Zur Verdoppelung der Anregungsintensität wird der transmittierte Lichtstrahl durch einen Spiegel reflektiert und nochmals durch die Probe gestrahlt.

Das Fluoreszenzlicht wird mittels optischer Elemente (Linsensystem) auf den Detektor, eine Silicium-Photodiode, abgebildet. Das rückseitig abgestrahlte Licht wird von einem Hohlspiegel ebenfalls auf die Silicium-Photodiode geworfen.

Zur Abtrennung des Störlichts (gestreutes Anregungslicht) vom Fluoreszenzlicht werden Kanten- und/oder Interferenzfilter und/ oder ein Polarisator (NIR-Polarisationsfolie) benutzt.

Die Optimierung des Polarisators wird dabei so gewählt, daß die Richtung der maximalen Transmission senkrecht zur Polarisationsebene des Anregungslichts steht.

Beispiel 25

Es wurde soviel Farbstoff der Formel

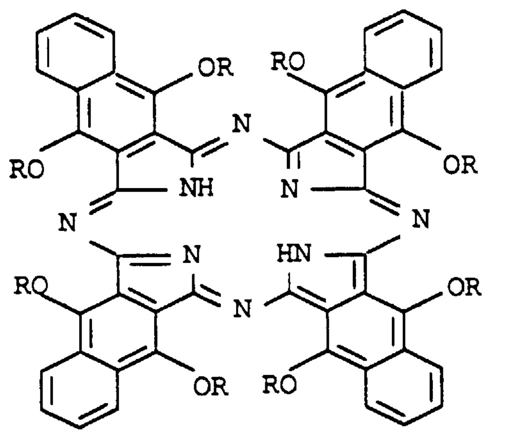

$R=n-C_4H_9$

in Dieselkraftstoff gelöst, daß eine Stammlösung mit einem Gehalt an Farbstoff von 219 ppb erhalten wurde. Weitere Lösungen wurden hieraus durch Verdünnen mit Dieselkraftstoff hergestellt.

Diese Lösungen wurden gemäß der allgemeinen Vorschrift II unter Anwendung der folgenden apparativen Größen vermessen.

Anregung: GaAlAs-Halbleiterdiodenlaser mit der Laserwellenlänge 813 nm; CW-Leistung 7 mW.

Filter: Langpaß-Interferenzfilter 850 nm (Fa. Corion).

Photodetektor: Silicium-Photodiode mit einer Fläche von 1 $cm^2$ (Fa. UDT). Der Photostrom wurde mit einem Strom/ Spannungs-Wandler (Fa. UDT, Modell 350) gemessen.

Die Ergebnisse sind in der folgenden Tabelle aufgeführt.

| Farbstoffgehalt im Dieselkraftstoff [ppb] | Extinktion bei $\lambda_{max}$ | Fluoreszenzsignal (in Skalenteilen) |
|---|---|---|
| 219 | 0,05 | 2366 |
| 43,7 | 0,01 | 451 |
| 8,75 | 0,002 | 106 |
| 1,75 | 0,0004 | 40 |
| 0 | 0,0 | 20 |

Damit gilt für den Nachweis des Markers mittels Fluoreszenz eine untere Nachweisgrenze von ca. 5 ppb.

Ähnlich günstige Ergebnisse werden erzielt, wenn man Naphthalocyanine der obengenannten Formel (mit $R = n-C_5H_{11}$ oder $n-C_{12}H_{25}$) oder die im folgenden aufgeführten Farbstoffe zum Markieren verwendet.

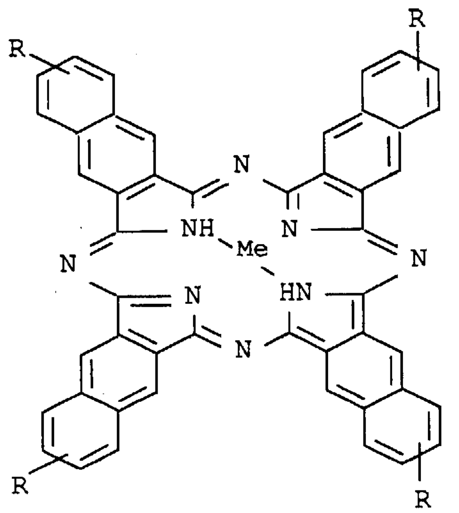

$(R=t\text{-}C_4H_9)$

Den Farbstoffen 26 bis 28 liegt jeweils die voranstehende Formel zugrunde.

Farbstoff 26

Me = 2H

Farbstoff 27

Me = Zn

Farbstoff 28

Me = AlCl

Farbstoff 29

$$NcSi[\text{-}O\text{-}Si(CH_3)_2\text{-}O\text{-}C_{12}H_{25}]_2$$

Farbstoff 30

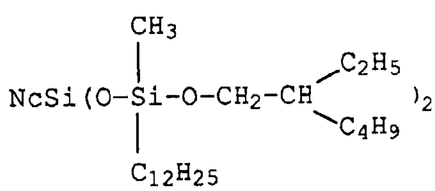

Farbstoff 31

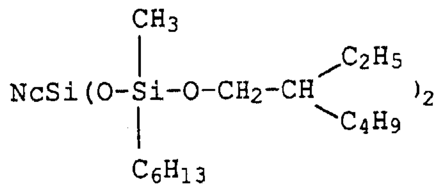

Farbstoff 32

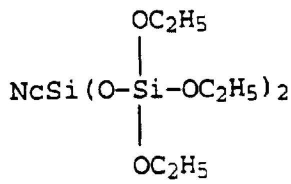

Farbstoff 33

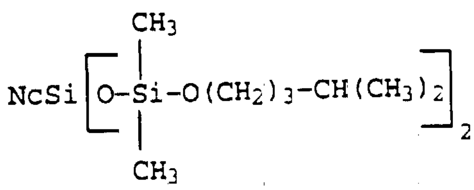

Farbstoff 34

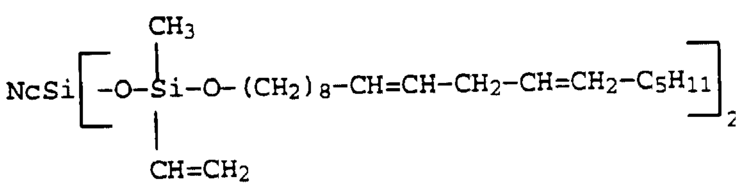

Farbstoffe 35 bis 37

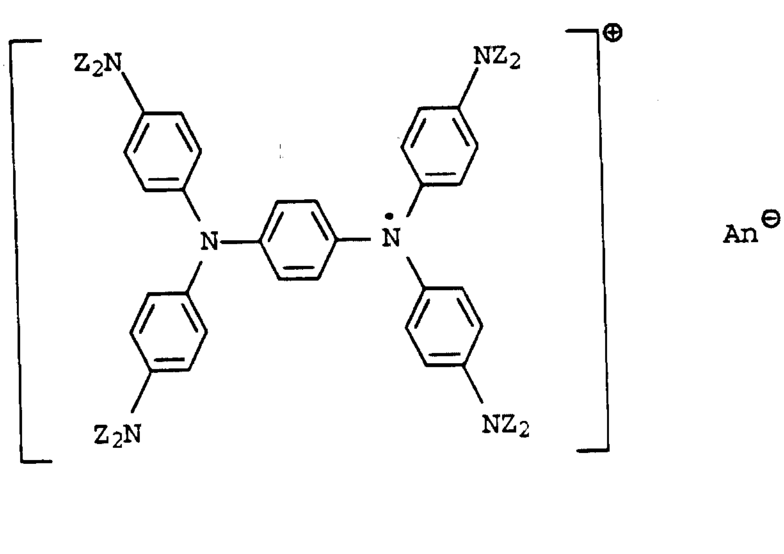

| Farbstoff Nr. | Z | $An^{\ominus}$ |
|---|---|---|
| 35 | $C_4H_9$ | $NO_3^{\ominus}$ |
| 36 | $C_2H_5$ | $NO_3^{\ominus}$ |
| 37 | $C_4H_9$ | $BF_4^{\ominus}$ |

Farbstoffe 38 bis 42

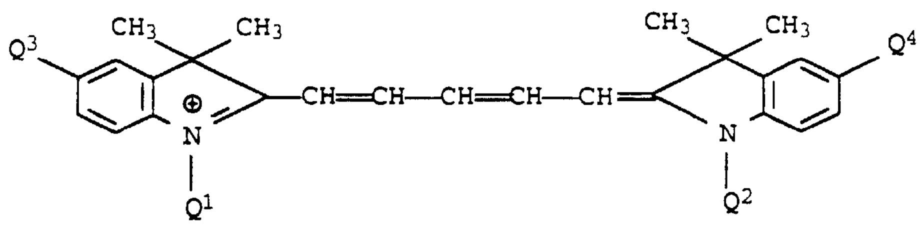

| Farbstoff Nr. | $Q^1$ | $Q^2$ | $Q^3$ | $Q^4$ | $An^{\ominus}$ |
|---|---|---|---|---|---|
| 38 | $CH_3$ | $CH_3$ | Cl | Cl | $I^{\ominus}$ |
| 39 | $CH_3$ | $CH_3$ | H | H | $I^{\ominus}$ |
| 40 | $C_2H_4OC(=O)NHC(CH_3)_3$ | $C_2H_4OC(=O)NHC(CH_3)_3$ | H | H | $I^{\ominus}$ |
| 41 | $C_2H_4C(=O)NHC_6H_{13}$ | $C_2H_4C(=O)NHC_6H_{13}$ | H | H | $ClO_4^{\ominus}$ |
| 42 | $C_3H_6SO_3^{\ominus}$ | $C_3H_6SO_3H$ | H | H | Betain |

Farbstoff Nr. 43

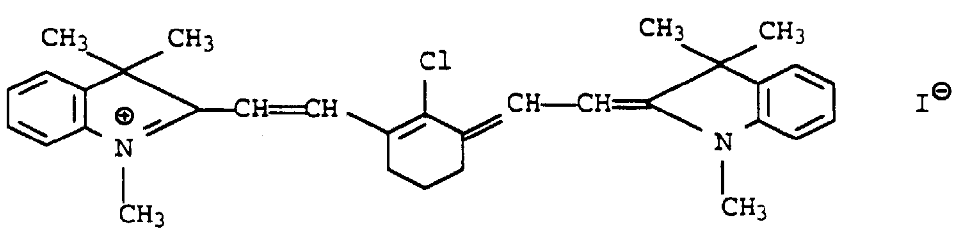

**Patentansprüche**

1. Verwendung von Verbindungen aus der Klasse der metallfreien oder metallhaltigen Phthalocyanine, der metallfreien oder metallhaltigen Naphthalocyanine, der Nickel-Dithiolen-Komplexe, der Aminiumverbindungen von aromatischen Aminen, der Methinfarbstoffe oder der Azulenquadratsäurefarbstoffe, die ihr Absorptionsmaximum im

Bereich von 600 bis 1 200 nm und/oder ein Fluoreszenzmaximum im Bereich von 620 bis 1 200 nm aufweisen, als Markierungsmittel für Flüssigkeiten.

**2.** Verwendung von Verbindungen nach Anspruch 1, dadurch gekennzeichnet, daß sie aus der Klasse der metallfreien oder metallhaltigen Naphthalocyanine oder der Nickel-Dithiolen-Komplexe stammen.

**3.** Verwendung von Verbindungen nach Anspruch 1, dadurch gekennzeichnet, daß sie aus der Klasse der metallfreien oder metallhaltigen Naphthalocyanine stammen.

**4.** Verfahren zur Detektion von Markierungsmitteln in Flüssigkeiten, dadurch gekennzeichnet, daß die Markierungsmittel anhand ihrer Fluoreszenz im NIR-Spektralbereich nachgewiesen werden.

**5.** Verfahren nach Anspruch 4, dadurch gekennzeichnet, daß die Fluoreszenz mit einem Halbleiterlaser oder einer Halbleiterdiode angeregt wird.

**6.** Verfahren nach Anspruch 4, dadurch gekennzeichnet, daß das Fluoreszenzlicht mit einem Halbleiterdetektor detektiert wird.

**7.** Verfahren nach Anspruch 4, dadurch gekennzeichnet, daß ein Halbleiterlaser oder eine Halbleiterdiode mit einer Wellenlänge der maximalen Emission im Spektralbereich von $\lambda_{max}$-100nm bis $\lambda_{max}$ + 20 nm verwendet wird, wobei $\lambda_{max}$ die Wellenlänge des Absorptionsmaximums des Markierungsmittels bezeichnet.

**8.** Verfahren nach Anspruch 4, dadurch gekennzeichnet, daß ein Halbleiterlaser oder eine Halbleiterdiode mit einer Wellenlänge der maximalen Emission von 620 bis 1200 nm verwendet wird.

**9.** Detektor zur Ausübung des Verfahrens nach Anspruch 4, dadurch gekennzeichnet, daß er eine NIR-Lichtquelle, ausgewählt aus einem Halbleiterlaser und einer Halbleiterdiode, ein oder mehrere optische Filter, einen NIR-Polarisator und einen Photodetektor, ausgewählt aus einer Silicium-Photodiode und einer Germanium-Photodiode, sowie gegebenenfalls Lichtleitfasern oder -faserbündel enthält.

**Claims**

**1.** The use of a compound from the class of metal-free or metal-containing phthalocyanines, metal-free or metal-containing naphthalocynanines, nickel dithiolene complexes, aminium compounds of aromatic amines, methine dyes or azulenesquaric acid dyes which have their absorption maximum in the range from 600 to 1,200 nm and/or a fluorescence maximum in the range from 620 to 1,200 nm, as a marker for liquids.

**2.** The use of a compound as claimed in claim 1, which comes from the class of metal-free or metal-containing naphthalocyanines or nickel dithiolene complexes.

**3.** The use of a compound as claimed in claim 1, which comes from the class of metal-free or metal-containing naphthalocyanines.

**4.** A method for detecting a marker in liquids, wherein the marker is detected from its fluorescence in the NIR spectral region.

**5.** A method as claimed in claim 4, wherein the fluorescence is excited by means of a semiconductor laser or a semiconductor diode.

**6.** A method as claimed in claim 4, wherein the fluorescence light is detected by means of a semiconductor detector.

**7.** A method as claimed in claim 4, wherein a semiconductor laser or a semiconductor diode having a wavelength of maximum emission in the spectral range from $\lambda_{max}$-100 nm to $\lambda_{max}$ +20 nm is used, where $\lambda_{max}$ is the wavelength of the absorption maximum of the marker.

**8.** A method as claimed in claim 4, wherein a semiconductor laser or a semiconductor diode having a wave-length of maximum emission of from 620 to 1,200 nm is used.

9. A detector for carrying out the method as claimed in claim 4, which comprises an NIR light source selected from a semiconductor laser and a semiconductor diode, one or more optical filters, an NIR polarizer and a photodetector selected from a silicon photodiode and a germanium photodiode and, if desired, optical fibers or optical fiber bundles.

**Revendications**

1. Utilisation de composés de la classe des phtalocyanines contenant ou non du métal, des naphtalocyanines contenant ou non du métal, des complexes nickel-dithiol, des composés aminium d'amines aromatiques, des colorants méthiniques ou des colorants d'acide azulènequadratique, qui présentent leur maximum d'absorption dans la plage de 600 à 1200 nm et/ou un maximum de fluorescence dans la plage de 620 à 1200 nm, comme agents de marquage pour liquides.

2. Utilisation de composés selon la revendication 1, caractérisés en ce qu'ils proviennent de la classe des naphtalocyanines contenant ou non du métal ou des complexes nickel-dithiol.

3. Utilisation de composés selon la revendication 1, caractérisés en ce qu'ils proviennent de la classe des naphtalocyanines contenant ou non du métal.

4. Procédé de détection d'agents de marquage dans des liquides, caractérisé en ce que la présence des agents de marquage est décelée au moyen de leur fluorescence dans le domaine spectral de l'infrarouge proche.

5. Procédé selon la revendication 4, caractérisé en ce que la fluorescence est excitée avec un laser à semiconducteur ou une diode à semiconducteur.

6. Procédé selon la revendication 4, caractérisé en ce que la lumière fluorescente est détectée avec un détecteur à semiconducteur.

7. Procédé selon la revendication 4, caractérisé en ce que l'on utilise un laser à semiconducteur ou une diode à semiconducteur ayant une longueur d'onde de l'émission maximale dans le domaine spectral de $\lambda_{max}$ - 100 nm à $\lambda_{max}$ + 20 nm, $\lambda_{max}$ désignant la longueur d'onde du maximum d'absorption de l'agent de marquage.

8. Procédé selon la revendication 4, caractérisé en ce que l'on utilise un laser à semiconducteur ou une diode à semiconducteur ayant une longueur d'onde de l'émission maximale de 620 à 1200 nm.

9. Détecteur pour l'exécution du procédé selon la revendication 4, caractérisé en ce qu'il contient une source de lumière infrarouge proche, choisie entre un laser à semiconducteur et une diode à semiconducteur, un ou plusieurs filtres optiques, un polariseur d'infrarouge proche et un photodétecteur, choisi entre une photodiode au silicium et une photodiode au germanium, ainsi éventuellement que des fibres optiques ou des faisceaux de fibres optiques.

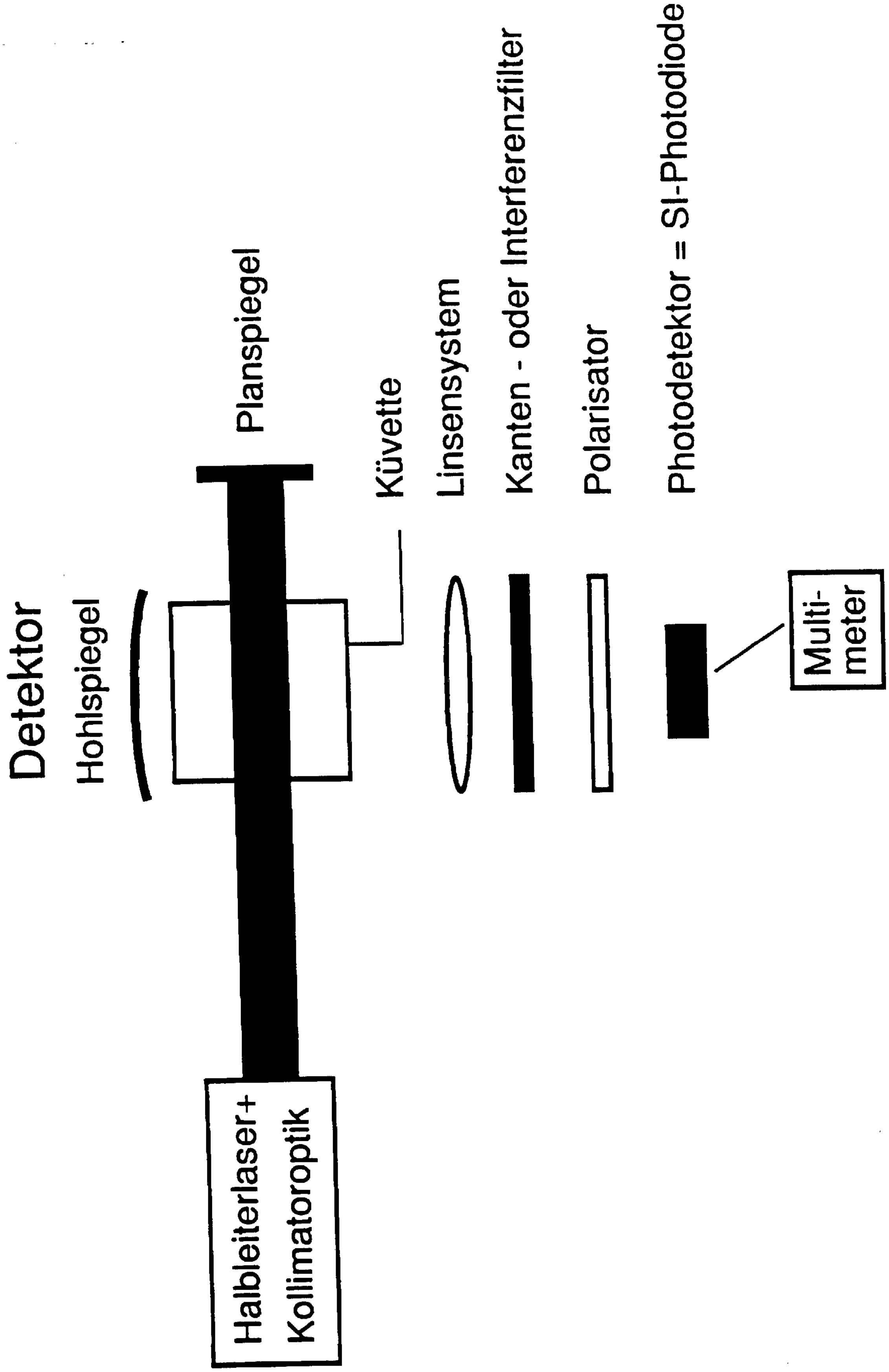
Detektor
Hohlspiegel
Planspiegel
Küvette
Linsensystem
Kanten - oder Interferenzfilter
Polarisator
Photodetektor = SI-Photodiode
Multi-
meter
Halbleiterlaser+
Kollimatoroptik